# EUROPÄISCHE PATENTSCHRIFT

(11) **EP 0 717 036 B1**
(45) Veröffentlichungstag und Bekanntmachung des Hinweises auf die Patenterteilung: **17.03.1999**
(21) Anmeldenummer: 95119053.7
(22) Anmeldetag: 04.12.1995
(51) Int. Cl.: C07D 211/82, A61K 31/44, C07D 211/90

(54) **Verwendung von 5-Acyl-1,4-dihydropyridin zur Bekämpfung der Erkrankungen des ZNS**
Use of 5-acyl-1,4-dihydropyridines for the treatment of diseases of the CNS
L'utilisation de 5-acyle-1,4-dihydropyridines pour le traitement des maladies du SNC

(30) Priorität: 16.12.1994 DE 4444864
(43) Veröffentlichungstag der Anmeldung: 19.06.1996
(73) Patentinhaber: BAYER AG, 51368 Leverkusen (DE)
(72) Erfinder: Urbahns, Klaus, Dr., D-42115 Wuppertal (DE); Heine, Hans-Georg, Dr., D-47800 Krefeld (DE); Glaser, Thomas, Dr., D-51491 Overath (DE); Wittka, Reilinde, Dr., D-51069 Köln (DE); De Vry, Jean-Marie-Viktor, Dr., D-51503 Rösrath (DE); Sommermeyer, Henning, Dr., D-51061 Köln (DE)

(56) Entgegenhaltungen:
- EP-A- 0 406 502
- EP-A- 0 675 409
- DE-A- 2 018 739
- DE-A- 2 302 866
- US-A- 3 966 948

## Beschreibung

Die vorliegende Erfindung betrifft die neue Verwendung von teilweise bekannten 1-Alkyl-3,5-diacyl-1,4-dihydropyridinen, Verfahren zu ihrer Herstellung und ihre Verwendung als Arzneimittel, als selektive Kaliumkanalmodulatoren, insbesondere zur Behandlung des zentralen Nervensystems.

Aus den Publikationen DOS 20 18 738 und 20 18 739 und US 39 66 948 sind Acyl-1,4-dihydropyridine mit Kreislaufwirkung bekannt.

Außerdem sind einige 4-Aryl-2,6-dimethyl-3,5-diacetyl-1,4-dihydropyridine als Synthesebausteine in der Publikation Chem. Het. Compounds (Engl. Transl.), Bd. 19, 1983, Heft 4, S. 415-419 = Khim. Geterosikl. Soedin Bd. 4, 1983, S. 508-513 beschrieben.

Es wurde nun gefunden, daß die teilweise bekannten 1-Alkyl-5-acyl-1,4-dihydropyridine der allgemeinen Formel (I) 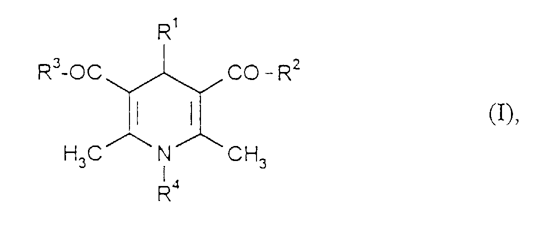 in welcher
R¹ für Phenyl steht, das gegebenenfalls bis zu 5-fach gleich oder verschieden durch Nitro, Cyano, Halogen, Trifluormethyl oder durch geradkettiges oder verzweigtes Alkylthio mit bis zu 6 Kohlenstoffatomen substituiert ist,
R² und R³ gleich oder verschieden sind und jeweils
   für geradkettiges oder verzweigtes Alkyl mit bis zu 8 Kohlenstoffatomen oder Phenyl stehen, oder
   - R²: für geradkettiges oder verzweigtes Alkoxy mit bis zu 8 Kohlenstoffatomen oder für Phenoxy steht, und
   - R⁴: für geradkettiges oder verzweigtes Alkyl mit bis zu 4 Kohlenstoffatomen stehen,
überraschenderweise eine selektive modulierende Wirkung auf Kaliumkanäle besitzen und geeignet sind zur Verwendung bei der Bekämpfung von Erkrankungen des zentralen Nervensystems und der Sichelzellenanämie.

Die erfindungsgemäßen Verbindungen können in stereoisomeren Formen existieren, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen.

Bevorzugt verwendet werden Verbindungen der allgemeinen Formel (I),
in welcher
- R¹: für Phenyl steht, die gegebenenfalls bis zu 3-fach gleich oder verschieden durch Nitro, Cyano, Fluor, Chlor, Brom, Iod, Trifluormethyl oder durch geradkettiges oder verzweigtes Alkylthio mit bis zu 4 Kohlenstoffatomen substituiert sind,
- R² und R³: gleich oder verschieden sind und jeweils für geradkettiges oder verzweigtes Alkyl mit bis zu 6 Kohlenstoffatomen oder Phenyl stehen, oder
- R²: für geradkettiges oder verzweigtes Alkoxy mit bis zu 6 Kohlenstoffatomen oder für Phenoxy steht, und
- R⁴: für geradkettiges oder verzweigtes Alkyl mit bis zu 4 Kohlenstoffatomen steht,
bei der Bekämpfung von Erkrankungen des zentralen Nervensystems.

Besonders bevorzugt verwendet werden Verbindungen der allgemeinen Formel (I),
in welcher
- R¹: für Phenyl steht, das gegebenenfalls bis zu 3-fach gleich oder verschieden durch Nitro, Cyano, Fluor, Chlor, Brom, Iod, Trifluormethyl oder durch Methylthio substituiert ist,
- R² und R³: gleich oder verschieden sind und jeweils für Alkyl mit bis zu 4 Kohlenstoffatomen oder für Phenyl stehen, oder
- R²: für Alkoxy mit bis zu 4 Kohlenstoffatomen oder für Phenoxy steht, und
- R⁴: für Methyl oder Ethyl steht,
bei der Bekämpfung von Erkrankungen des zentralen Nervensystems.

Die erfindungsgemäßen Verbindungen der allgemeinen Formel (I) zeigen ein nicht vorhersehbares, wertvolles pharmakologisches Wirkspektrum.

Sie sind Kanalmodulatoren mit einer überraschenden Selektivität für calciumabhängige Kalium-Kanäle großer Leitfähigkeit (BK(Ca)-Kanäle), insbesondere die Kalium-Kanäle des zentralen Nervensystems.

Aufgrund ihrer pharmakologischen Eigenschaften können sie für die Herstellung von Arzneimitteln zur Behandlung von zentral degenerativen Erkrankungen eingesetzt werden, wie z.B. bei Auftreten von Demenzen (Multiinfarktdemenz (MID), primär degenerativer Demenz (PDD), prä- und seniler Alzheimerscher Krankheit, HIV-Demenz und andere Demenzformen), Parkinsonscher Krankheit oder amyotrophischer Lateralsklerose sowie multipler Sklerose.

Weiterhin eignen sich die Wirkstoffe zur Behandlung von Hirnleistungsstörungen im Alter, des hirnorganischen Psychosyndroms (HOPS, Organic Brain Syndrom, OBS) und von altersbedingten Gedächtnisstörungen (age associated memory impairment, AAMI).

Sie sind geeignet zur Prophylaxe, Behandlung und zur Bekämpfung der Folgen cerebraler Durchblutungsstörungen wie cerebraler Ischämien, Schlaganfällen, Schädel-Hirn-Traumata und von Subarachnoidalblutungen.

Sie sind wertvoll zur Behandlung von Depressionen und Psychosen, z.B. Schizophrenie. Außerdem eignen sie sich zur Behandlung von Störungen der neuroendokrinen Sekretion sowie der Neurotransmittersekretion und damit zusammenhängenden gesundheitlichen Störungen wie Manie, Alkoholismus, Drogenmißbrauch, Sucht oder krankhaftem Eßverhalten. Weitere Anwendungsgebiete sind die Behandlung von Migräne, Schlafstörungen und von Neuropathien. Darüber hinaus sind sie als Schmerzmittel geeignet.

Die Wirkstoffe sind ferner geeignet zur Behandlung von Störungen des Immunsystems, insbesondere der T-Lymphocyten-Proliferation und zur Beeinflussung der glatten Muskulatur, insbesondere von Uterus, Harnblase und Bronchialtrakt und zur Behandlung damit zusammenhängender Krankheiten wie z.B. Asthma und urinärer Inkontinenz und zur Behandlung von Arrhythmie, Angina und Diabetes.

Außerdem betrifft die Erfindung neue ausgewählte Verbindungen der allgemeinen Formel (I), mit den in der folgenden Tabelle angegebenen Substituentenbedeutungen:

| **R**^{**1**} | **R**^{**2**} | **R**^{**3**} | **R**^{**4**} |
|---|---|---|---|
| 2,4,5-Cl-C₆H₂ | CH₃ | CH₃ | CH₃ |
| 2,3,5-Cl-C₆H₂ | CH₃ | CH₃ | CH₃ |
| 3,4,5-F₃-C₆H₂ | CH₃ | CH₃ | CH₃ |
| 2,3-Cl-C₆H₃ | CH₃ | CH₃ | CH₃ |
| 4-Cl-C₆H₄ | CH₃ | CH₃ | CH₃ |
| 3,4-Cl-C₆H₃ | CH₃ | CH₃ | CH₃ |
| 4-F-C₆H₄ | CH₃ | CH₃ | CH₃ |
| 4-Cl-C₆H₄ | OCH₃ | -CH₃ | -CH₃ |
| 2,3-Cl-C₆H₃ | OCH₃ | -CH₃ | -CH₃ |
| | | | |
| 3,4,5-F-C₆H₂ | OCH₃ | -CH₃ | -CH₃ |
| 4-F-C₆H₄ | OCH₃ | -CH₃ | -CH₃ |
| 3,4-Cl-C₆H₃ | OCH₃ | -CH₃ | -CH₃ |
| 4-NO₂-C₆H₄ | OCH₃ | -CH₃ | -CH₃ |
| 3-CF₃, 4-Cl-C₆H₂ | OCH₃ | -CH₃ | -CH₃ |

Die erfindungsgemäßen Verbindungen der Formel (I) können hergestellt werden, indem man
A) Aldehyde der allgemeinen Formel (II)

   R¹-CHO (II)

   in welcher
   - R¹: die oben angegebene Bedeutung hat,
   mit β-Ketoverbindungen der allgemeinen Formel (III) 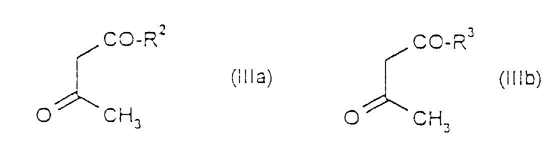 in welcher
   - R² und R³: die oben angegebene Bedeutung haben,
   und mit Alkylaminhydrochloriden in inerten Lösemitteln, gegebenenfalls in Anwesenheit einer Base, umsetzt, oder
B) Aldehyde der Formel (II) mit β-Ketoverbindungen der Formel (IIIa) und Enaminen der Formel (IV) 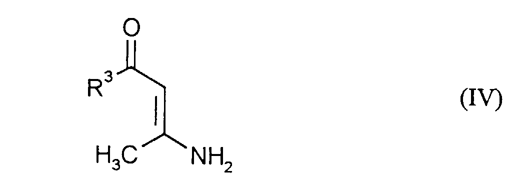 in welcher
   - R³: die oben angegebene Bedeutung hat,
   zunächst zu 1,4-Dihydropyridinen der allgemeinen Formel (V) 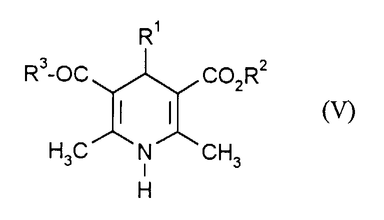 in welcher
   - R¹, R² und R³: die oben angegebene Bedeutung haben,
   in inerten Lösemitteln umsetzt,
   und anschließend mit Alkylierungsmitteln der allgemeinen Formel (VI)

   R⁴-L (VI)

   in welcher
   - R⁴: die oben angegebene Bedeutung hat und
   - L: für Halogen, vorzugsweise für Brom oder Iod steht,
   gegebenenfalls unter Schutzgasatmosphäre in inerten Lösemitteln und in Gegenwart einer Base umsetzt.

Die erfindungsgemäßen Verfahren können durch folgendes Formelschema beispielhaft erläutert werden:

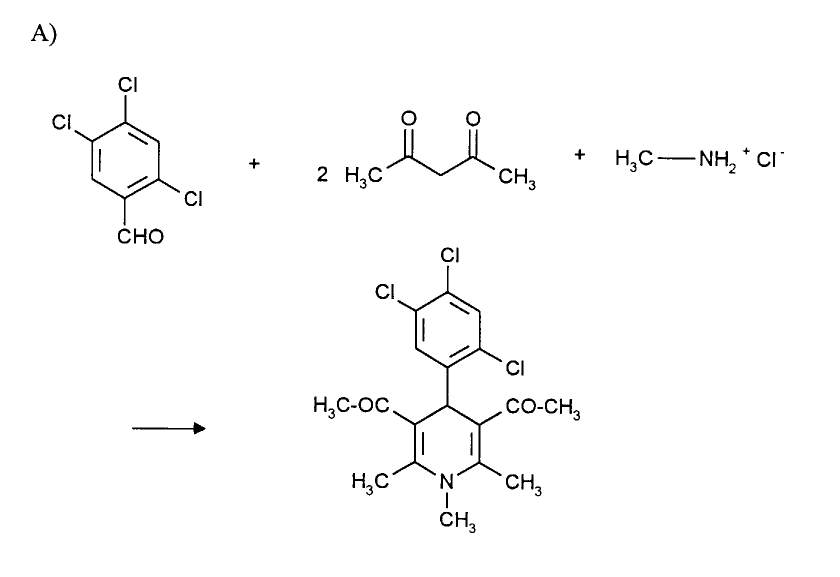

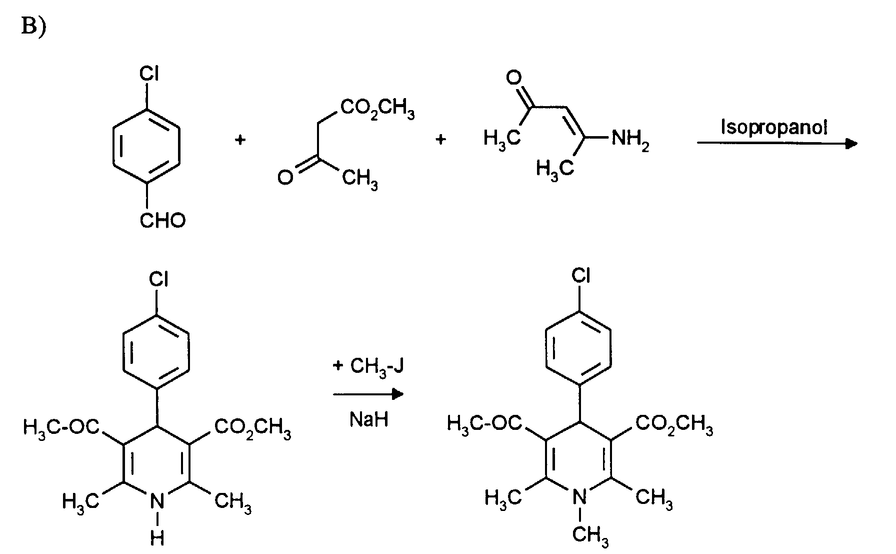

Als Lösemittel eignen sich alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether, Acetonitril, Aceton oder Amide wie Hexamethylphosphorsäuretriamid oder Dimethylformamid, oder halogenierte Kohlenwasserstoffe wie Methylenchlorid, Tetrachlorkohlenstoff oder Kohlenwasserstoffe wie Benzol oder Toluol oder Pyridin. Ebenso ist es möglich, Gemische der genannten Lösemittel zu verwenden. Bevorzugt ist für das Verfahren (A) Pyridin, für das Verfahren (B) Isopropanol.

Die Reaktionstemperaturen können in einem größeren Bereich variiert werden. Im allgemeinen arbeitet man zwischen +10°C und +150°C, vorzugsweise zwischen +20°C und +100°C, insbesondere bei der Siedetemperatur des jeweiligen Lösemittels.

Die Umsetzungen können bei Normaldruck, aber auch bei erhöhtem oder erniedrigtem Druck (z.B. 0,5 bis 3 bar) durchgeführt werden. Im allgemeinen arbeitet man bei Normaldruck.

Für die Alkylierung eignen sich im allgemeinen übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt ist Dimethylformamid.

Als Basen für die Alkylierung eignen sich im allgemeinen Alkalialkoholate wie Natrium- oder Kaliummethanolat, Natrium- oder Kaliumethanolat oder Kalium-tert.-butylat. Es ist auch möglich, als Basen Alkalimetalle wie Natrium oder deren Hydride wie Natriumhydrid einzusetzen. Bevorzugt ist Natriumhydrid.

Die Alkylierung erfolgt im allgemeinen mit Alkylierungsmitteln wie beispielsweise (C₁-C₄)-Alkylhalogeniden, Sulfonsäureestern oder substituierten oder unsubstituierten (C₁-C₄)-Dialkyl-sulfate, vorzugsweise Methyliodid oder Dimethylsulfat.

Die Alkylierung erfolgt im allgemeinen in einem der oben aufgeführten Lösemitteln, vorzugsweise in Dimethylformamid in einem Temperaturbereich von 0°C bis +70°C, vorzugsweise von 0°C bis +30°C und Normaldruck.

Bei der Durchführung der erfindungsgemäßen Verfahren ist das Verhältnis der an der Reaktion beteiligten Stoffe beliebig. Im allgemeinen arbeitet man jedoch bei molaren Mengen der Reaktanden.

Die Verbindungen der allgemeinen Formeln (II), (III), (IV) und (V) sind bekannt oder können nach bekannten Methoden hergestellt werden.

Enantiomerenreine Formen erhält man z.B., indem man Diastereomerengemische der Verbindungen der allgemeinen Formel (I), in welcher R² für einen optisch aktiven Esterrest steht, nach üblicher Methode trennt, anschließend entweder direkt umestert oder zuerst die chiralen Carbonsäuren herstellt und dann durch Veresterung die enantiomerenreinen Dihydropyridine herstellt.

Die Trennung der Diastereomeren erfolgt im allgemeinen entweder durch fraktionierte Kristallisation, durch Säulenchromatographie oder durch Craig-Verteilung. Welches das optimale Verfahren ist, muß von Fall zu Fall entschieden werden, manchmal ist es auch zweckmäßig, Kombinationen der einzelnen Verfahren zu benutzen. Besonders geeignet ist die Trennung durch Kristallisation oder Craig-Verteilung bzw. eine Kombination beider Verfahren.

Die enantiomerenreinen Verbindungen sind u.a. zugänglich durch Chromatographie der racemischen Ester auf chiralen Phasen.

Die erfindungsgemäßen Verbindungen der allgemeinen Formel (I) zeigen ein nicht vorhersehbares Wirkspektrum, insbesondere auf Grund ihrer Selektivität auf calciumabhängige Kalium-Kanäle großer Leitfähigkeit.

### ⁸⁶Rubidium-Efflux aus C6-BU1-Glioma-Zellen

Die Versuche wurden mit geringfügigen Veränderungen entsprechend der von Tas et al. (Neurosci. Lett. 94, 279-284, (1988)) beschriebenen Methode durchgeführt. Dazu werden C6-BU1-Glioma-Zellen aus Ratten verwendet. Aus den durch Flüssigkeitszintillation erhaltenen Daten wird die durch Ionomycin hervorgerufene Erhöhung des Effluxes über den Basalefflux berechnet und als 100 % gesetzt. Die Stimulationen in Gegenwart von Prüfsubstanzen werden dann auf diesen Wert bezogen.

Zur vorliegenden Erfindung gehören auch pharmazeutische Zubereitungen, die neben inerten, nicht-toxischen, pharmazeutisch geeigneten Hilfs- und Trägerstoffen eine oder mehrere Verbindungen der allgemeinen Formel (I) enthalten, oder die aus einem oder mehreren Wirkstoffen der Formel (I) bestehen, sowie Verfahren zur Herstellung dieser Zubereitungen.

Die Wirkstoffe der Formel (I) sollen in diesen Zubereitungen in einer Konzentration von 0,1 bis 99,5 Gew.-%, bevorzugt von 0,5 bis 95 Gew.-% der Gesamtmischung vorhanden sein.

Neben den Wirkstoffen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, den oder die Wirkstoffe der Formel (I) in Gesamtmengen von etwa 0,01 bis etwa 100 mg/kg, bevorzugt in Gesamtmengen von etwa 1 mg/kg bis 50 mg/kg Körpergewicht je 24 Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und vom Körpergewicht des behandelten Objekts, vom individuellen Verhalten gegenüber dem Medikament, der Art und Schwere der Erkrankung, der Art der Zubereitung und Applikation, sowie dem Zeitpunkt bzw. Intervall, zu welchem die Verabreichung erfolgt.

### Ausgangsverbindungen

### Beispiel I

### 5-Acetyl-2,6-dimethyl-4-(4-chlorphenyl)-1,4-dihydropyridin-3-carbonsäuremethylester

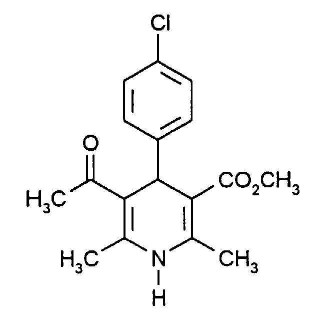

5,64 g (40 mmol) 4-Chlorbenzaldehyd, 4,0 g (40 mmol) 4-Aminopent-3-en-2-on sowie 4,6 g (40 mmol) Methylacetoacetat werden in 100 ml Isopropanol 12 h zum Rückfluß erhitzt. Man läßt abkühlen und engt die Reaktionsmischung ein. Aus Et₂O kristallisieren 1,85 g der Titelverbindung.

### Herstellungsbeispiele

### Beispiel 1

### 1-[5-Acetyl-4-(2,4,5-trichlorphenyl)-1,2,6-trimethyl-1,4-dihydropyridin-3-yl]ethanon

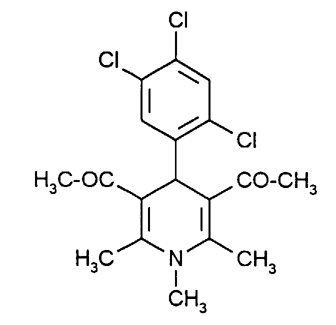

5,0 g (23,9 mmol) 2,4,5-Trichlorbenzaldehyd, 4,8 g (47,8 mmol) Acetylaceton und 1,78 g (26,3 mmol) Methylamin-hydrochlorid werden in 4 ml Pyridin 5 h am Rückfluß gekocht. Anschließend wird das Pyridin abgezogen und der Rückstand zweimal mit Toluol kodestilliert. Man nimmt in AcOEt auf und extrahiert mit 1 N wäßriger HCl. Trocknen und Einengen der wäßrigen Phase liefert ein braunes Öl, das durch Flashchromatographie gereinigt wird (Petrolether / AcOEt = 5:1). Abschließend wird aus Ether umkristallisiert. Man erhält 4,2 g der Titelverbindung (45% d.Th.).
MS: 385
R_{f} = 0,65 (Petrolether : AcOEt = 1:1)

In Analogie zur Vorschrift des Beispiels 1 werden die in Tabelle 1 aufgeführten Verbindungen hergestellt:

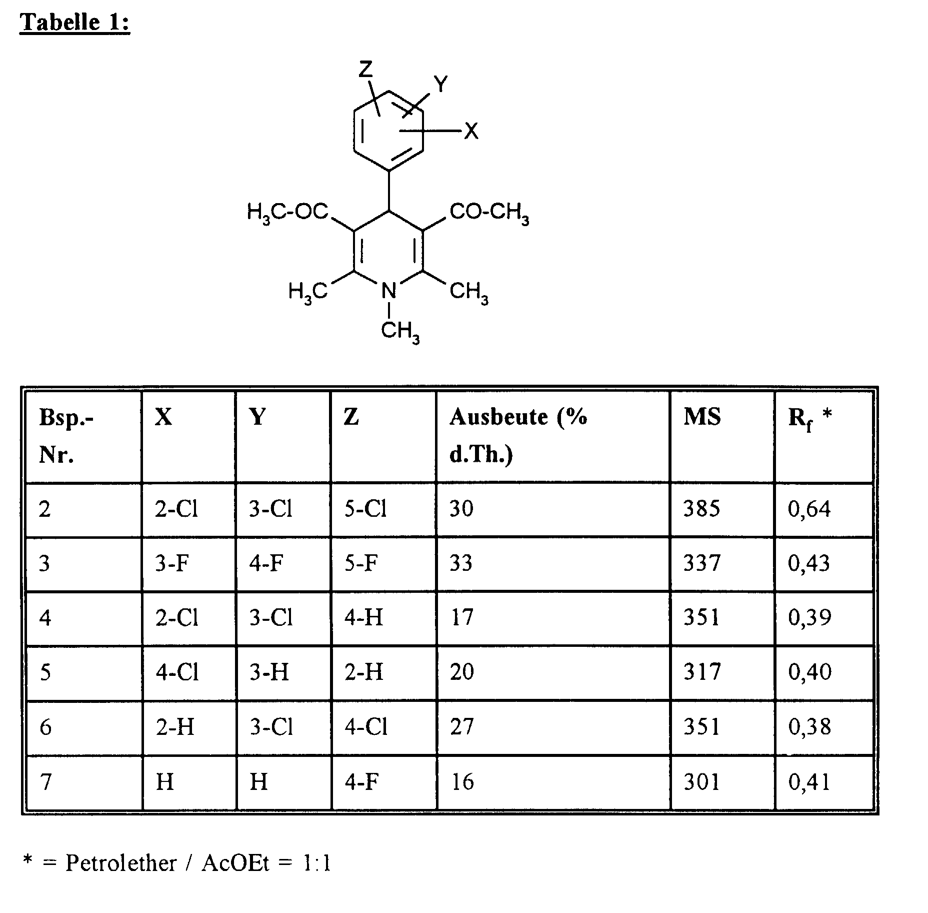

### Beispiel 8

### 5-Acetyl-4-(4-chlorphenyl)-1,2,6-trimethyl-1,4-dihydropyridin-3-carbonsäuremethylester

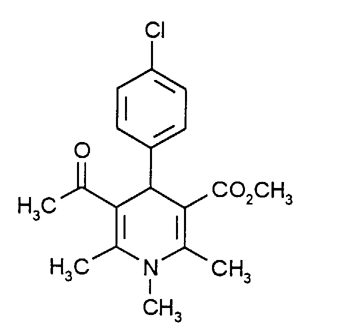

1,0 g (3,2 mmol) der Verbindung aus Beispiel I werden in 15 ml DMF gelöst und unter Argon mit 180 mg NaH versetzt. Man läßt 15 Minuten bei 0°C rühren. Dann tropft man 0,51 ml (6,2 mmol) MeI hinzu und rührt erneut 30 min. Man versetzt nacheinander mit H₂O und Essigsäureethylester und wäscht die organische Phase mit gesättigter wäßriger NaCl-Lösung. Dann wird eingeengt und über Kieselgel aufgetrennt (Petrolether/AcOEt = 1+1). Die entsprechenden Fraktionen kristallisieren aus Et₂O/Petrolether. Man erhält 230 mg der Titelverbindung.
MS: 333,8
R_{f} = 0,57 (Petrolether/AeOEt = 1+1)

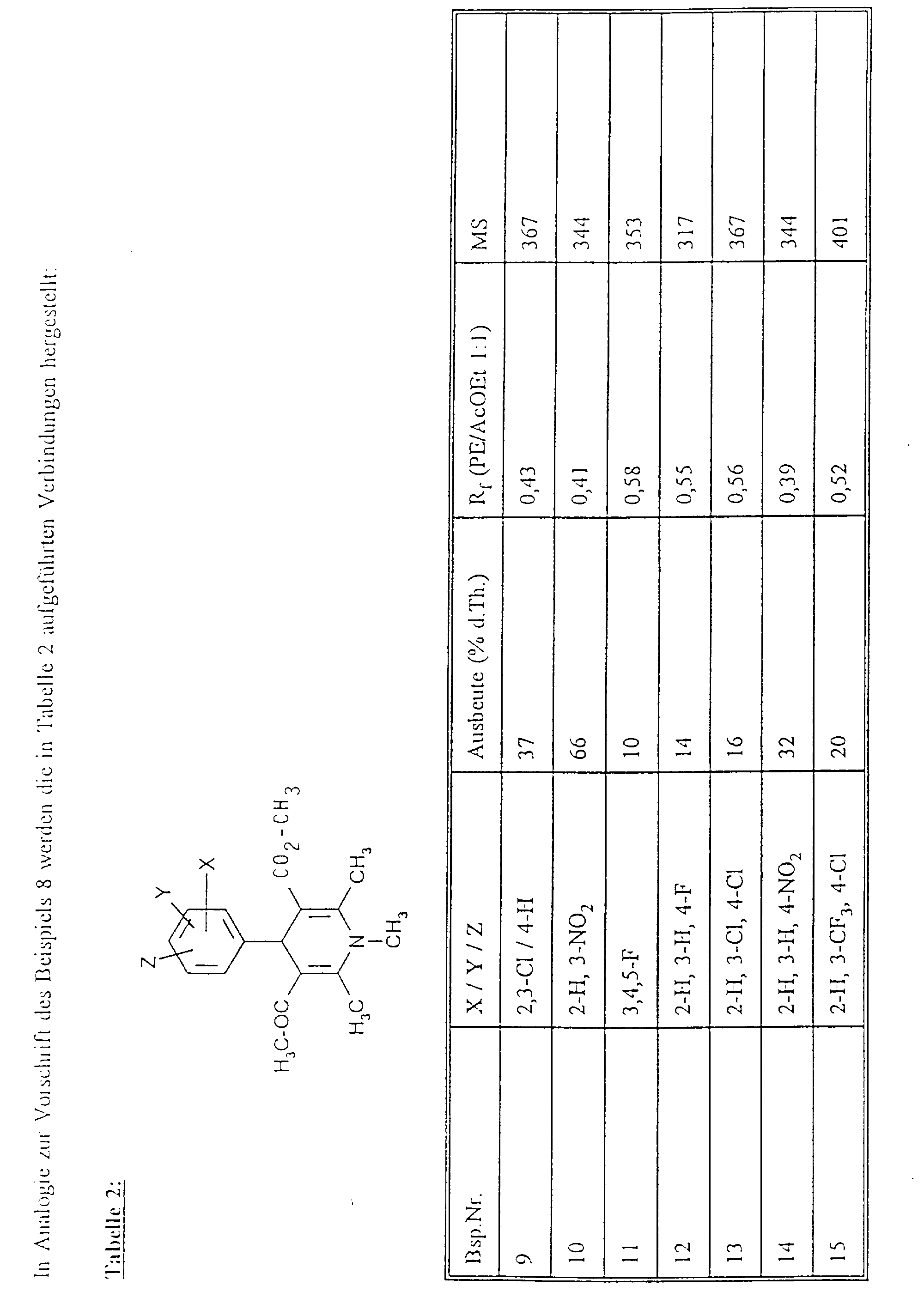

## Patentansprüche

1. Verwendung von 1-Alkyl-5-acyl-1,4-dihydropyridinen der allgemeinen Formel (I) 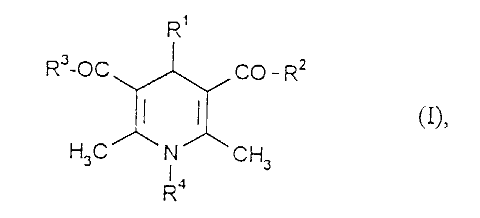 in welcher
R¹ für Phenyl steht, das gegebenenfalls bis zu 5-fach gleich oder verschieden durch Nitro, Cyano, Halogen, Trifluormethyl oder durch geradkettiges oder verzweigtes Alkylthio mit bis zu 6 Kohlenstoffatomen substituiert ist,
R² und R³ gleich oder verschieden sind und jeweils für geradkettiges oder verzweigtes Alkyl mit bis zu 8 Kohlenstoffatomen oder Phenyl stehen, oder
R² für geradkettiges oder verzweigtes Alkoxy mit bis zu 8 Kohlenstoffatomen oder für Phenoxy steht, und
R⁴ für geradkettiges oder verzweigtes Alkyl mit bis zu 4 Kohlenstoffatomen stehen,
zur Herstellung von Arzneimitteln zur Bekämpfung von Erkrankungen des zentralen Nervensystems, die eine selektiv modulierende Wirkung auf Kaliumkanäle besitzen.

2. Verwendung von Verbindungen der allgemeinen Formel (I) gemäß Anspruch 1, in welcher
R¹ für Phenyl steht, die gegebenenfalls bis zu 3-fach gleich oder verschieden durch Nitro, Cyano, Fluor, Chlor, Brom, Iod, Trifluormethyl oder durch geradkettiges oder verzweigtes Alkylthio mit bis zu 4 Kohlenstoffatomen substituiert sind,
R² und R³ gleich oder verschieden sind und jeweils für geradkettiges oder verzweigtes Alkyl mit bis zu 6 Kohlenstoffatomen oder Phenyl stehen, oder
R² für geradkettiges oder verzweigtes Alkoxy mit bis zu 6 Kohlenstoffatomen oder für Phenoxy steht, und
R⁴ für geradkettiges oder verzweigtes Alkyl mit bis zu 4 Kohlenstoffatomen steht,
zur Herstellung von Arzneimitteln gemäß Anspruch 1.

3. Verwendung von Verbindungen der allgemeinen Formel (I) gemäß Anspruch 1, in welcher
R¹ für Phenyl steht, das gegebenenfalls bis zu 3-fach gleich oder verschieden durch Nitro, Cyano, Fluor, Chlor, Brom, Iod, Trifluormethyl oder durch Methylthio substituiert ist,
R² und R³ gleich oder verschieden sind und jeweils für Alkyl mit bis zu 4 Kohlenstoffatomen oder für Phenyl stehen, oder
R² für Alkoxy mit bis zu 4 Kohlenstoffatomen oder für Phenoxy steht, und
R⁴ für Methyl oder Ethyl steht,
zur Herstellung von Arzneimitteln gemäß Anspruch 1.

4. Verbindungen der allgemeinen Formel (I) gemäß Anspruch 1, ausgewählt aus der folgenden Gruppe
1-[5-Acetyl-4-(2,4,5-trichlorphenyl)-1,2,6-trimethyl-1-,4-dihydropyridin-3-yl]ethanon
1-[5-Acetyl-4-(2,3,5-trichlorphenyl)-1,2,6-trimethyl-1,4-dihydropyridin-3-yl]ethanon
1-[5-Acetyl-4-(3,4,5-trichlorphenyl)-1,2,6-trimethyl-1,4-dihydropyridin-3-yl]ethanon
1-[5-Acetyl-4-(2,3-dichlorphenyl)-1,2,6-trimethyl-1,4-dihydropyridin-3-yl]ethanon
1-[5-Acetyl-4-(4-chlorphenyl)-1,2,6-trimethyl-1,4-dihydropyridin-3-yl]ethanon
1-[5-Acetyl-4-(3,4-dichlorphenyl)-1,2,6-trimethyl-1,4-dihydropyridin-3-yl]ethanon
1-[5-Acetyl-4-(4-fluorphenyl)-1,2,6-trimethyl-1,4-dihydropyridin-3-yl]ethanon
5-Acetyl-4-(4-chlorphenyl)-1,2,6-trimethyl-1,4-dihydropyridin-3-carbonsäure-methylester
5-Acetyl-4-(2,3-dichlorphenyl)-1,2,6-trimethyl-1,4-dihydropyridin-3-carbonsäure-methylester
5-Acetyl-4-(3,4,5-trifluorphenyl)-1,2,6-trimethyl-1,4-dihydropyridin-3-carbonsäure-methylester
5-Acetyl-4-(4-fluorphenyl)-1,2,6-trimethyl-1,4-dihydropyridin-3-carbonsäure-methylester
5-Acetyl-4-(3,4-dichlorphenyl)-1,2,6-trimethyl-1,4-dihydropyridin-3-carbonsäure-methylester
5-Acetyl-4-(4-nitrophenyl)-1,2,6-trimethyl-1,4-dihydropyridin-3-carbonsäure-methylester
5-Acetyl-4-(4-chlor-3-trifluormethylphenyl)-1,2,6-trimethyl-1,4-dihydropyridin-3-carbonsäure-methylester

5. Verfahren zur Herstellung von Verbindungen der allgemeinen Formel (I) gemäß Anspruch 1 und von Verbindungen gemäß Anspruch 4, dadurch gekennzeichnet, daß man
A) Aldehyde der allgemeinen Formel (II)
R¹-CHO (II)
in welcher
R¹ die oben angegebene Bedeutung hat,
mit β-Ketoverbindungen der allgemeinen Formel (III) 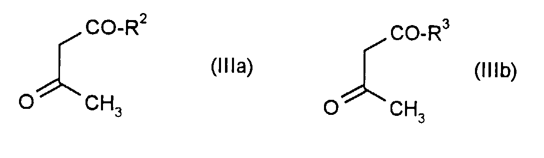 in welcher
R² und R³ die oben angegebene Bedeutung haben,
und mit Alkylaminhydrochloriden in inerten Lösemitteln, gegebenenfalls in Anwesenheit einer Base, umsetzt, oder
B) Aldehyde der Formel (II) mit β-Ketoverbindungen der Formel (IIIa) und Enaminen der Formel (IV) 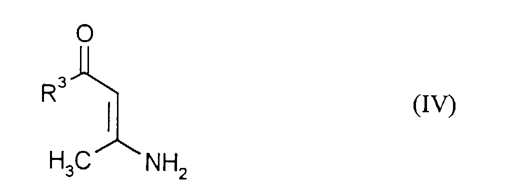 in welcher
R³ die oben angegebene Bedeutung hat,
zunächst zu 1,4-Dihydropyridinen der allgemeinen Formel (V) 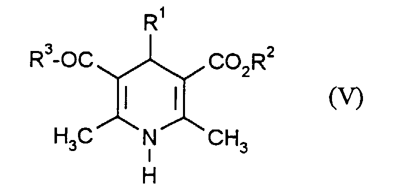 in welcher
R¹, R² und R³ die oben angegebene Bedeutung haben,
in inerten Lösemitteln umsetzt,
und anschließend mit Alkylierungsmitteln der allgemeinen Formel (VI)
R⁴-L (VI)
in welcher
R⁴ die oben angegebene Bedeutung hat und
L für Halogen, vorzugsweise für Brom oder Iod steht,
gegebenenfalls unter Schutzgasatmosphäre in inerten Lösemitteln und in Gegenwart einer Base umsetzt.

6. Arzneimittel zur Behandlung des zentralen Nervensystems enthaltend mindestens eine Verbindung der allgemeinen Formel (I) gemäß Anspruch 1 als selektiven Kaliumkanalmodulator.

7. Verfahren zur Herstellung von Arzneimitteln, dadurch gekennzeichnet, daß man mindestens eine Verbindung der allgemeinen Formel (I) gemäß Anspruch 1 gegebenenfalls unter Verwendung von üblichen Hilfs- und Trägerstoffen in eine geeignete Applikationsform überführt.

## Claims

1. Use of 1-alkyl-5-acyl-1,4-dihydropyridines of the general formula (I) 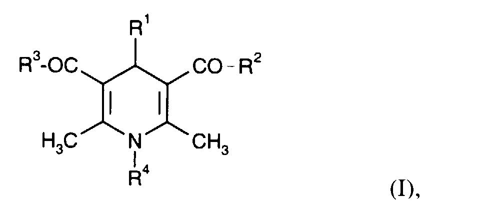 in which
R¹ represents phenyl, which is optionally substituted up to 5 times by identical or different nitro, cyano, halogen or trifluoromethyl substituents or by straight-chain or branched alkylthio having up to 6 carbon atoms,
R² and R³ are identical or different and each represent straight-chain or branched alkyl having up to 8 carbon atoms or phenyl, or
R² represents straight-chain or branched alkoxy having up to 8 carbon atoms or phenoxy, and
R⁴ represents straight-chain or branched alkyl having up to 4 carbon atoms,
for the production of medicaments for the control of disorders of the central nervous system, which have a selective modulating action on potassium channels.

2. Use of compounds of the general formula (I) according to Claim 1, in which
R¹ represents phenyl or naphthyl, each of which is optionally substituted up to 3 times by identical or different nitro, cyano, fluorine, chlorine, bromine, iodine or trifluoromethyl substituents or by straight-chain or branched alkylthio having up to 4 carbon atoms,
R² and R³ are identical or different and each represent straight-chain or branched alkyl having up to 6 carbon atoms or phenyl, or
R² represents straight-chain or branched alkoxy having up to 6 carbon atoms or phenoxy, and
R⁴ represents straight-chain or branched alkyl having up to 4 carbon atoms,
for the production of medicaments according to Claim 1.

3. Use of compounds of the general formula (I) according to Claim 1, in which
R¹ represents phenyl which is optionally substituted up to 3 times by identical or different nitro, cyano, fluorine, chlorine, bromine, iodine or trifluoromethyl substituents or by methylthio,
R² and R³ are identical or different and each represent alkyl having up to 4 carbon atoms or phenyl, or
R² represents alkoxy having up to 4 carbon atoms or phenoxy, and
R⁴ represents methyl or ethyl,
for the production of medicaments according to Claim 1.

4. Compounds of the general formula (I) according to Claim 1, selected from the following group
1-[5-Acetyl-4-(2,4,5-trichlorophenyl)-1,2,6-trimethyl-1,4-dihydropyridin-3-yl]-ethanone
1-[5-Acetyl-4-(2,3 5-trichlorophenyl)-1,2,6-trimethyl-1,4-dihydropyridin-3-yl]-ethanone
1-[5-Acetyl-4-(3,4,5-trichlorophenyl)-1,2,6-trimethyl-1,4-dihydropyridin-3-yl]-ethanone
1-[5-Acetyl-4-(2,3-dichlorophenyl)-1,2,6-trimethyl-1,4-dihydropyridin-3-yl]-ethanone
1-[5-Acetyl-4-(4-chlorophenyl)-1,2,6-trimethyl-1,4-dihydropyridin-3-yl]-ethanone
1-[5-Acetyl-4-(3,4-dichlorophenyl)-1,2,6-trimethyl-1,4-dihydropyridin-3-yl]-ethanone
1-[5-Acetyl-4-(4-fluorophenyl)-1,2,6-trimethyl-1,4-dihydropyridin-3-yl]-ethanone
Methyl 5-acetyl-4-(4-chlorophenyl)-1,2,6-trimethyl-1,4-dihydropyridine-3-carboxylate
Methyl 5-acetyl-4-(2,3-dichlorophenyl)-1,2,6-trimethyl-1,4-dihydro-pyridine-3-carboxylate
Methyl 5-acetyl-4-(3,4,5-trifluorophenyl)-1,2,6-trimethyl-1,4-dihydropyridine-3-carboxylate
Methyl 5-acetyl-4-(4-fluorophenyl)-1,2,6-trimethyl-1,4-dihydropyridine-3-carboxylate
Methyl 5-acetyl-4-(3,4-dichlorophenyl)-1,2,6-trimethyl-1,4-dihydropyridine-3-carboxylate
Methyl 5-acetyl-4-(4-nitrophenyl)-1,2,6-trimethyl-1,4-dihydropyridine-3-carboxylate
Methyl 5-acetyl-4-(4-chloro-3-trifluoromethylphenyl)-1,2,6-trimethyl-1,4-dihydropyridine-3-carboxylate

5. Process for the preparation of compounds of the general formula (I) according to Claim 1 and of compounds according to Claim 4, characterized in that
A) aldehydes of the general formula (II)
R¹-CHO (II)
in which
R¹ has the meaning indicated above,
are reacted with -keto compounds of the general formula (III) 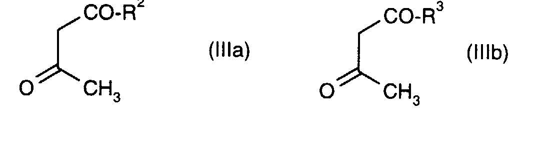 in which
R² and R³ have the meaning indicated above,
and with alkylamine hydrochlorides in inert solvents, if appropriate in the presence of a base, or
B) aldehydes of the formula (II) are reacted with -keto compounds of the formula (IIIa) and enamines of the formula (IV) 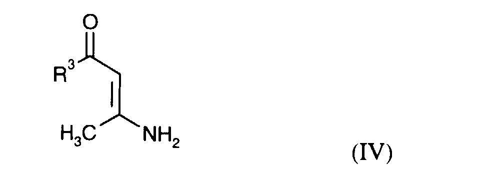 in which
R³ has the meaning indicated above,
first to give 1,4-dihydropyridines of the general formula (V) 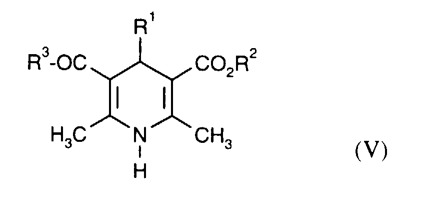 in which
R¹, R² and R³ have the meaning indicated above,
in inert solvents,
and these are then reacted with alkylating agents of the general formula (VI)
R⁴-L (VI)
in which
R⁴ has the meaning indicated above and
L represents halogen, preferably bromine or iodine,
if appropriate under a protective gas atmosphere, in inert solvents and in the presence of a base.

6. Medicaments for the treatment of the central nervous system, containing at least one compound of the general formula (I) according to Claim 1 as a selective potassium channel modulator.

7. Process for the production of medicaments, characterized in that at least one compound of the general formula (I) according to Claim 1 is converted into a suitable administration form, if appropriate using customary auxiliaries and excipients.

## Revendications

1. Utilisation de 1-alkyl-5-acyl-1,4-dihydropyridines répondant à la formule générale (I) 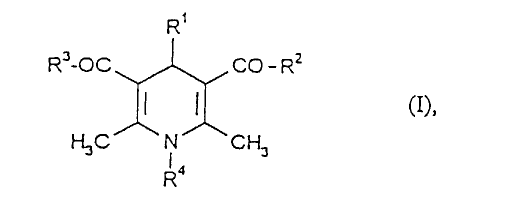 dans laquelle
R¹ représente un groupe phényle qui porte, le cas échéant, jusqu'à 5 substituants identiques ou différents nitro, cyano, halogéno, trifluorométhyle ou alkylthio à chaîne droite ou ramifiée contenant jusqu'à 6 atomes de carbone,
R² et R³ sont identiques ou différents et représentent respectivement un groupe alkyle à chaîne droite ou ramifiée contenant jusqu'à 8 atomes de carbone, ou un groupe phényle, ou bien
R² représente un groupe alcoxy à chaîne droite ou ramifiée contenant jusqu'à 8 atomes de carbone, ou un groupe phénoxy, et
R⁴ représente un groupe alkyle à chaîne droite ou ramifiée contenant jusqu'à 4 atomes de carbone,
pour la préparation de médicaments pour lutter contre des maladies du système nerveux central, qui ont un effet modulateur de manière sélective sur les canaux du potassium.

2. Utilisation de composés répondant à la formule générale (I) selon la revendication 1, dans laquelle
R¹ représente un groupe phényle qui porte, le cas échéant, jusqu'à 3 substituants identiques ou différents nitro, cyano, fluoro, chloro, bromo, iodo, trifluorométhyle ou alkylthio à chaîne droite ou ramifiée contenant jusqu'à 4 atomes de carbone,
R² et R³ sont identiques ou différents et représentent respectivement un groupe alkyle à chaîne droite ou ramifiée contenant jusqu'à 6 atomes de carbone, ou un groupe phényle, ou bien
R² représente un groupe alcoxy à chaîne droite ou ramifiée contenant jusqu'à 6 atomes de carbone, ou un groupe phénoxy, et
R⁴ représente un groupe alkyle à chaîne droite ou ramifiée contenant jusqu'à 4 atomes de carbone,
pour la préparation de médicaments selon la revendication 1.

3. Utilisation de composés répondant à la formule générale (I) selon la revendication 1, dans laquelle
R¹ représente un groupe phényle qui porte, le cas échéant, jusqu'à 3 substituants identiques ou différents nitro, cyano, fluoro, chloro, bromo, iodo, trifluorométhyle ou méthylthio,
R² et R³ sont identiques ou différents et représentent respectivement un groupe alkyle à chaîne droite ou ramifiée contenant jusqu'à 4 atomes de carbone, ou un groupe phényle, ou bien
R² représente un groupe alcoxy contenant jusqu'à 4 atomes de carbone, ou un groupe phénoxy, et
R⁴ représente un groupe méthyle ou une groupe éthyle,
pour la préparation de médicaments selon la revendication 1.

4. Composés répondant à la formule générale (I) selon la revendication 1, choisis parmi le groupe comprenant les composés ci-après:
la 1-[5-acétyl-4-(2,4,5-trichlorophényl)-1,2,6-triméthyl-1,4-dihydropyridin-3-yl]éthanone
la 1-[5-acétyl-4-(2,3,5-trichlorophényl)-1,2,6-triméthyl-1,4-dihydropyridin-3-yl]éthanone
la 1-[5-acétyl-4-(3,4,5-trichlorophényl)-1,2,6-triméthyl-1,4-dihydropyridin-3-yl]éthanone
la 1-[5-acétyl-4-(2,3-dichlorophényl)-1,2,6-triméthyl-1,4-dihydropyridin-3-yl]éthanone
la 1-[5-acétyl-4-(4-chlorophényl)-1,2,6-triméthyl-1,4-dihydropyridin-3-yl]éthanone
la 1-[5-acétyl-4-(3,4-dichlorophényl)-1,2,6-triméthyl-1,4-dihydropyridin-3-yl]éthanone
la 1-[5-acétyl-4-(4-fluorophényl)-1,2,6-triméthyl-1,4-dihydropyridine-3-yl]éthanone
l'ester méthylique de l'acide 5-acétyl-4-(4-chlorophényl)-1,2,6-triméthyl-1,4-dihydropyridine-3-carboxylique
l'ester méthylique de l'acide 5-acétyl-4-(2,3-dichlorophényl)-1,2,6-triméthyl-1,4-dihydropyridin-3-carboxylique
l'ester méthylique de l'acide 5-acétyl-4-(3,4,5-trifluorophényl)-1,2,6-triméthyl-1,4-dihydropyridine-3-carboxylique
l'ester méthylique de l'acide 5-acétyl-4-(4-fluorophényl)-1,2,6-triméthyl-1,4-dihydropyridine-3-carboxylique
l'ester méthylique de l'acide 5-acétyl-4-(3,4-dichlorophényl)-1,2,6-triméthyl-1,4-dihydropyridine-3-carboxylique
l'ester méthylique de l'acide 5-acétyl-4-(4-nitrophényl)-1,2,6-triméthyl-1,4-dihydropyridine-3-carboxylique
l'ester méthylique de l'acide 5-acétyl-4-(4-chloro-3-trifluorométhylphényl)-1,2,6-triméthyl-1,4-dihydropyridine-3-carboxylique.

5. Procédé pour la préparation de composés répondant à la formule générale (I) selon la revendication 1 et des composés selon la revendication 4, caractérisé en ce qu'on fait réagir
A) des aldéhydes répondant à la formule générale (II)
R¹-CHO (II)
dans laquelle
R¹ a la signification indiquée ci-dessus,
avec des composés β-cétoniques répondant à la formule générale (III) 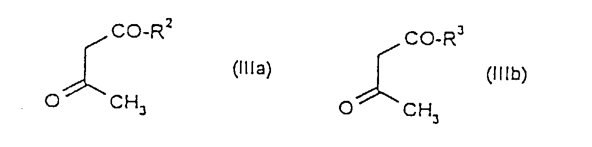 formules dans lesquelles
R² et R³ ont la signification indiquée ci-dessus,
et avec des chlorhydrates d'alkylamines dans des solvants inertes, le cas échéant en présence d'une base, ou bien en ce qu'on fait d'abord réagir
B) des aldéhydes de formule (II) avec des composés β-cétoniques de formule (IIIa) et des énamines de formule (IV) 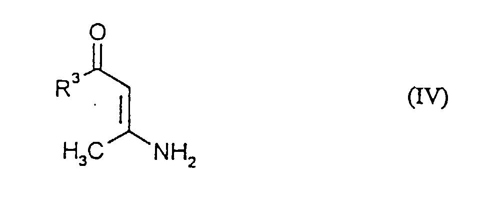 dans laquelle
R³ a la signification indiquée ci-dessus,
pour obtenir des 1,4-dihydropyridines répondant à la formule générale (V) 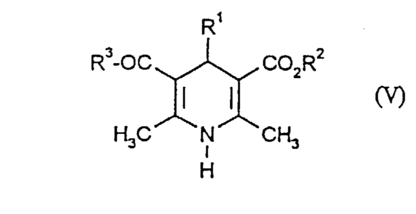 dans laquelle
R¹, R² et R³ ont la signification indiquée ci-dessus,
dans des solvants inertes,
et en ce qu'on les fait ensuite réagir avec des agents d'alkylation répondant à la formule générale (VI)
R⁴-L (VI)
dans laquelle
R⁴ a la signification indiquée ci-dessus, et
L représente un atome d'halogène, de préférence un atome de brome ou un atome d'iode,
le cas échéant, sous l'atmosphère d'un gaz de protection, dans des solvants inertes et en présence d'une base.

6. Médicaments pour le traitement du système nerveux central, contenant au moins un composé répondant à la formule générale (I) selon la revendication 1, à titre de modulateur sélectif du canal du potassium.

7. Procédé pour la préparation de médicaments caractérisé en ce qu'on transforme au moins un composé répondant à la formule générale (I) selon la revendication 1 , le cas échéant en utilisant des substances auxiliaires et de support habituelles, en une forme d'application appropriée.
